# EUROPEAN PATENT SPECIFICATION

(11) **EP 1 290 129 B1**
(45) Date of publication and mention of the grant of the patent: **09.01.2008**
(21) Application number: 01938007.0
(22) Date of filing: 05.06.2001
(51) Int. Cl.: C11D 3/386, D06M 16/00, D06P 5/15

(54) **REDEPOSITION OR BACKSTAIN INHIBITION DURING STONEWASHING PROCESS**
VERHINDERUNG DER WIEDERABLAGERUNG ODER RÜCKANSCHMUTZUNG WÄHREND DES STONE-WASH-PROCESSES
INHIBITION DE LA REDEPOSITION OU DE LA RECOLORATION AU COURS D'UN PROCEDE DE DELAVAGE A LA PIERRE

(30) Priority: 02.06.2000 DK 200000861; 23.10.2000 DK 200001577; 24.11.2000 DK 200001772; 19.01.2001 DK 200100100
(43) Date of publication of application: 12.03.2003
(73) Proprietor: Novozymes A/S, 2880 Bagsvaerd (DK)
(72) Inventor: UYAMA, Naoto, c/o Novozymes Japan Ltd., Seoul, Chiba 261-01 (JP); DAIMON, Kosaku, c/o Novozymes Jaoan Ltd., Seoul, Chiba 261-01 (JP)
(74) Representative: Shenker, Paul Dhan
(86) International application number: PCT/DK2001/000384
(87) International publication number: WO 2001/092453

(56) References cited:
- WO-A-94/07983
- WO-A-97/04160
- WO-A-99/51808
- US-A- 5 912 157

## Description

The present invention relates to methods for reducing or preventing the backstaining of dye on textile materials, especially indigo on denim and specially the backstaining of pocket parts of denim during the stonewashing of denim fabric.

### BACKGROUND OF THE INVENTION

By stonewashing of denim the usually blue-dyed denim is given a faded or worn appearance with the characteristic white and blue contrast. Stonewashing the denim material is typically carried out in the presence of purmice stone or cellulase or a combination thereof and results in the removal of dye to give areas of lighter colour. The use of cellulase instead of purmice stone has the advantages that it is more environmental friendly, more economical and prevents that the denim is damage because of the rough treatment with the purmice stones. However, the use of cellulase is not without disadvantages.

The dye removed from the denim material after the treatment with cellulase or by a conventional washing process may cause "backstaining" or "redeposition" onto the denim material, e.g. re-colouration of the blue threads and blue coloration of the white threads, resulting in a less contrast between the blue and white threads. In order to remove the dye the denim manufactures are using huge amount of surfactants to make parts white again at a soaping process with heavy washing condition. The heavy washing condition causes colour change or colour-fading problems for finished denim products. Also additional water has to be used in the subsequent soaping process.

The problem of redeposition or backstaining of dye during stonewashing has also been addressed by adding anti-redeposition chemicals, such as surfactants or other agents into the cellulase wash. Also the use of different cellulases with less specific activity on denim has been tried. WO-A-9407983 describes the use of a cellulase to inhibit the backstaining of denim. WO-A-9429426 and WO-A-9325655 describes backstain inhibition by treatment with a redoposition cellulase composition and added protease as an improvement over the use of redeposition cellulase alone.

Although, these methods aim to solve the problem with the backstaining or redeposition of dye onto the denim material, they may still be improved. Especially, the backstaining or redeposition of dye onto the pocket parts of the denim material pose a problem.

### SUMMARY OF THE INVENTION

We have developed a process for treating fabric, especially indigo-dyed denim, with a composition comprising a lipolytic enzyme.

This treatment reduces the risk of back-staining (redeposition of dye onto textile) even when less water is used. The enzymatic treatment of released dyestuff will decrease process time as well as the amount of energy and water needed to achieve a satisfactory quality of the textile, and the colour of the wastewater is reduced.

The method of the invention can result in a decreased number of washes, thereby increasing the productivity and decreasing the consumption of water and chemicals, including surfactants.

Accordingly, the present invention provides a method for reducing the backstaining of fabric or textile selected from the group consisting of polyamide fibres, acrylic fibres, and polyester fibres, comprising contacting the fabric or textile with a composition comprising an effective amount of a cutinase (EC 3.1.1.74).

In another aspect, the present invention relates to a method for reducing the backstaining of fabric or textile, comprising contacting the fabric or textile with a composition comprising a microbial cutinase (EC 3.1.1.74).

In a third aspect, the invention relates to the use of a composition comprising a cutinase (EC 3.1.1.74) and a cellulase for reducing backstaining of fabric or textile.

### DETAILED DISCLOSURE OF THE INVENTION

Denim that is stonewashed with the addition of an effective amount of added lipolytic enzyme during cellulase treatment shows a reduction in the level of backstaining, especially the backstaining of pocket parts.

The method of the present invention comprises contacting the denim to be enzymatically stonewashed with a composition comprising the lipolytic enzyme in a amount sufficient to reduce backstaining and thus, to decrease the blue-colouring of e.g. the pocket parts.

The amount of added lipolytic enzyme depends upon others on the purity and amount of cellulase used in the stonewashing process, the contact time, the amount of dye removed during stonewashing, the activity of the cellulase, the pH and temperature of the stonewashing process, the formulation of the product and the like.

The composition to be added may further comprise various adjuvants as known to the skilled person, e.g. surfactants. Other materials can also be used with the composition as desired, including stones, fillers, solvents, buffers, pH control agents, enzyme activators, builders, enzyme stabilizers, other anti-deposition agent and the like. The composition may be formulated at a solid product, granular product or as a liquid product.

The lipolytic enzyme may be added to the composition containing the cellulase for use in stonewashing process or added directly to the stonewashing bath or to a subsequent rinse treatment. The lipolytic enzyme may also be added to a composition for washing purposes thereby reducing or inhibiting the backstaining of removed dye during the washing process.

### Fabrics

The process of the present invention applies to fabrics in general. In the context of this invention fabrics include fabrics or textiles prepared from man-made fibers, e.g. polyester, nylon, etc., as well as cellulosic fabrics or textiles.

The term "cellulosic fabric/textile" indicates any type of fabric, in particular woven fabric, prepared from a cellulose-containing material, containing cellulose or cellulose derivatives, e.g. from wood pulp, and cotton. The main part of the cellulose or cellulose derivatives present on the fabric is normally size with which the yarns, normally warp yarns, have been coated prior to weaving. In the present context, the term "fabric" is also intended to include garments and other types of processed fabrics. Examples of cellulosic fabric is cotton, viscose (rayon); lyocell; all blends of viscose, cotton or lyocell with other fibers such as polyester; viscose/cotton blends, lyocell/cotton blends, viscose/wool blends, lyocell/wool blends, cotton/wool blends; flax (linen), ramie and other fabrics based on cellulose fibers, including all blends of cellulosic fibers with other fibers such as wool, polyamide, acrylic and polyester fibers, e.g. viscose/cotton/polyester blends, wool/cotton/polyester blends, flax/cotton blends etc. The fabric may also include man-made fibers alone such as polyester fibers.

The process of the invention is preferably applied to cellulose-containing fabrics, such as cotton, viscose, rayon, ramie, linen or mixtures thereof, or mixtures of any of these fibers with synthetic fibers. In particular, the fabric may be denim. The fabric may be dyed with vat dyes such as indigo, direct dyes such as Direct Red 185, sulfur dyes such as Sulfur Green 6, or reactive dyes fixed to a binder on the fabric surface. In a preferred embodiment of the present process, the fabric is indigo-dyed denim, including clothing items manufactured therefrom.

In a most preferred embodiment, the fabric subjected to the process of the invention is made of hydrophobic fibres such as polyamide fibres, e.g. nylon, acrylic fibres, vinylon and polyester fibres. As mention above the fabric may be made of mixtures of different fibres. Especially contemplated is polyester or polyester/cotton mixtures, which are the material used for pocket parts of garments, in particular dyed cotton garments or denim jeans.

### Enzyme

The enzymatic process of the invention is accomplished using a cutinase as classified in EC 3.1.1.74. Such enzymes are well known and defined in the literature, cf. e.g. Borgström B and Brockman H L, (Eds.); Lipases; Elsevier Science Publishers B.V., 1984, and Kolattukudy P E; The Biochemistry of Plants, Academic Press Inc., 1980 4 624-631.

The cutinase enzyme preferably is of microbial origin, in particular of bacterial, of fungal or of yeast origin.

In a particular embodiment, the cutinase enzyme used may be derived from a strain of *Absidia,* in particular *Absidia blakesleena* and *Absidia corymbifera,* a strain of *Achromobacter,* in particular *Achromobacter iophagus,* a strain of *Aeromonas,* a strain of *Alternaria,* in particular *Alternaria brassiciola,* a strain of *Aspergillus,* in particular *Aspergillus niger* and *Aspergillus flavus,* a strain of *Achromobacter*, in particular *Achromobacter iophagus,* a strain of *Aureobasidium,* in particular *Aureobasidium pullulans,* a strain of *Bacillus,* in particular *Bacillus pumilus, Bacillus strearothermophilus* and *Bacillus subtilis,* a strain of *Beauveria,* a strain of *Brochothrix,* in particular *Brochothrix thermosohata*, a strain of *Candida,* in particular *Candida cylindracea (Candida rugosa), Candida paralipolytica, Candida tsukubaensis, Candida auriculariae, Candida humicola, Cadida foliarum, Candida cylindracea (Cadida rugosa)* and *Candida antarctica,* a strain of *Chromobacter,* in particular *Chromobacter viscosum,* a strain of *Coprinus,* in particular *Coprinus cinerius,* a strain of *Fusarium,* in particular *Fusarium oxysporum, Fusarium solani, Fusarium solani pisi,* and *Fusarium roseum culmorum,* a strain of *Geotricum,* in particular *Geotricum penicillatum,* a strain of *Hansenula,* in particular *Hansenula anomala,* a strain of *Humicola,* in particular *Humicola brevispora, Humicula lanuginosa, Humicola brevis* var. thermoidea, and *Humicola insolens,* a strain of *Hyphozyma,* a strain of *Lactobacillus,* in particular *Lactobacillus curvatus,* a strain of *Metarhizium,* a strain of *Mucor,* a strain of *Paecilomyces,* a strain of *Penicillium,* in particular *Penicillium cyclopium, Penicillium crustosum* and *Penicillium expansum,* a strain of *Pseudomonas* in particular *Pseudomonas aeruginosa, Pseudomonas alcaligenes, Pseudomonas cepacia* (syn. *Burkholderia cepacia), Pseudomonas fluorescens, Pseudomonas fragi, Pseudomonas maltophilia, Pseudomonas mendocina, Pseudomonas mephitica lipolytica, Pseudomonas alcaligenes, Pseudomonas plantari, Pseudomonas pseudoalcaligenes, Pseudomonas putida, Pseudomonas stutzeri,* and *Pseudomonas wisconsinensis,* a strain of *Rhizoctonia,* in particular *Rhizoctonia solani,* a strain of *Rhizomucor,* in particular *Rhizomucor miehei,* a strain of *Rhizopus,* in particular *Rhizopus japonicus, Rhizopus microsporus* and *Rhizopus nodosus,* a strain of *Rhodosporidium,* in particular *Rhodosporidium toruloides,* a strain of *Rhodotorula,* in particular *Rhodotorula glutinis,* a strain of *Sporobolomyces,* in particular *Sporobolomyces shibatanus,* a strain of *Thermomyces,* in particular *Thermomyces lanuginosus* (formerly *Humicola lanuginosa),* a strain of *Thiarosporella,* in particular *Thiarosporella phaseolina,* a strain of *Trichoderma,* in particular *Trichoderma harzianum,* and *Trichoderma reesei,* and/or a strain of *Verticillium.*

In a more preferred embodiment, the cutinase enzyme used according to the invention is derived from a strain of *Aspergillus,* a strain of *Achromobacter,* a strain of *Bacillus, a* strain of *Candida,* a strain of *Chromobacter,* a strain of *Fusarium,* a strain of *Humicola,* a strain of *Hyphozyma,* a strain of *Pseudomonas,* a strain of *Rhizomucor,* a strain of *Rhizopus,* or a strain of *Thermomyces.*

In a more preferred embodiment, the cutinase enzyme used according to the invention is derived from a strain of *Bacillus pumilus,* a strain of *Bacillus stearothermophilus* a strain of *Candida cylindracea,* a strain of *Candida antarctica,* in particular *Candida antarctica* Lipase B (obtained as described in WO 88/02775), a strain of *Humicola insolens,* a strain of *Hyphozyma,* a strain of *Pseudomonas cepacia,* or a strain of *Thermomyces lanuginosus.*

In the context of this invention, a cutinase is an enzyme capable of degrading cutin, cf. e.g. Lin T S & Kolattukudy P E, J. Bacteriol. 1978 133 (2) 942-951, a suberinase is an enzyme capable of degrading suberin, cf. e.g. , Kolattukudy P E; Science 1980 208 990-1000, Lin T S & Kolattukudy P E; Physiol. Plant Pathol. 1980 17 1-15, and The Biochemistry of Plants, Academic Press, 1980 Vol. 4 624-634, and a poly-3-hydroxyalkanoate depolymerase is an enzyme capable of degrading poly-3-hydroxyalkanoate, cf. e.g. Foster et al., FEMS Microbiol. Lett. 1994 118 279-282. Cutinases, for instance, differs from classical lipases in that no measurable activation around the critical micelle concentration (CMC) of the tributyrine substrate is observed. Also, cutinases are considered belonging to a class of serine esterases.

The cutinase enzyme preferably is of microbial origin, in particular of bacterial, of fungal or of yeast origin.

In a preferred embodiment, the cutinase enzyme is derived from a strain of *Aspergillus,* in particular *Aspergillus oryzae,* a strain of *Alternaria,* in particular *Alternaria brassiciola,* a strain of Fusarium, in particular *Fusarium solani, Fusarium solani pisi, Fusarium roseum culmorum,* or *Fusarium roseum sambucium,* a strain of *Helminthosporum,* in particular *Helminthosporum sativum,* a strain of *Humicola,* in particular *Humicola insolens,* a strain of *Pseudomonas,* in particular *Pseudomonas mendocina,* or *Pseudomonas putida,* a strain of *Rhizoctonia,* in particular *Rhizoctonia solani,* a strain of *Streptomyces,* in particular *Streptomyces scabies,* or a strain of *Ulocladium,* in particular *Ulocladium consortiale.* In a most preferred embodiment the cutinase is derived from a strain of *Humicola insolens,* in particular the strain *Humicola insolens* DSM 1800.

Examples of cutinases include *Fusarium solani* lipase (cutinase) from Unilever.

### Process conditions

In the case of denim textiles (especially indigo-dyed denim), the process according to the invention can be carried out simultaneously with a treatment with cellulase (and optionally pumice) to create a desired worn look by forming local variations in colour density, as described in American dye stuff reporter, Sept. 90, D. Kochavi, T. Videbæk and D. Cedroni, Optimizing processing conditions in enzymatic stone washing. The process of the invention can also be carried out simultaneously with enzymatic desizing, i.e. removal of starch size by means of an ?-amylase. In a further aspect, the process is a conventional washing process, wherein the enzyme of the invention is added to a conventional detergent composition.

The process of the invention may be carried out at conventional conditions in a washing machine conventionally used for stone-washing, e.g. a washer-extractor. The enzyme of the invention should be added in an effective amount. By the term "effective amount" is meant the amount sufficient to reduce backstaining as compared to the backstaining effect when not applying the enzyme of the invention. Typical conditions are a temperature of 40-60 °C and a pH of 4.5-7.5. However, the process conditions must be chosen according to the characteristics of the enzyme in question. They are generally in the range 20-100?C, pH 4.5-10.5, typically 30-90?C, pH 4.5-7.5 especially 40-60?C, pH 4.5-6.5. Optionally, conventional additives may be used, e.g. a buffer, a surfactant (anionic and/or non-ionic) and/or a polymer (such as PVP, polyacrylate and polyacrylamide).

### MATERIALS AND METHODS

Enzymes:
Cutinase A (Cutinase variant from *Humicola Insolens* according to US 5,827,719).
Cutinase B (Cutinase variant from *Humicola Insolens* according to US 5, 827, 719).
Denimax^{®} 362S (available from Novozymes A/S).
Lipolase^{®} (available from Novozymes A/S).
Lipolase^{™} Ultra (available from Novozymes A/S).
Cellusoft^{®} L (available from Novozymes A/S)

### Lipolytic Activity

The lipolytic activity may be determined using tributyrine as substrate. This method is based on the hydrolysis of tributyrine by the enzyme, and the alkali consumption is registered as a function of time.

One Lipase Unit (LU) is defined as the amount of enzyme which, under standard conditions (i.e. at 30.0°C; pH 7.0; with Gum Arabic as emulsifier and tributyrine as substrate) liberates 1 mmol titrable butyric acid per minute (1 KLU = 1000 LU).

A folder AF 95/5 describing this analytical method in more detail is available upon request to Novozymes A/S, Denmark, which folder is hereby included by reference.

### Cellulytic Activity

The cellulytic activity may be measured in endo-glucanase units (EGU), determined at pH 6.0 with carboxymethyl cellulose (CMC) as substrate.

A substrate solution is prepared, containing 34.0 g/l CMC (Hercules 7 LFD) in 0.1 M phosphate buffer at pH 6.0. The enzyme sample to be analyzed is dissolved in the same buffer. 5 ml substrate solution and 0.15 ml enzyme solution are mixed and transferred to a vibration viscosimeter (e.g. MIVI 3000 from Sofraser, France), thermostated at 40°C for 30 minutes.

One EGU is defined as the amount of enzyme that reduces the viscosity to one half under these conditions. The amount of enzyme sample should be adjusted to provide 0.01-0.02 EGU/ml in the reaction mixture. The arch standard is defined as 880 EGU/g.

The cellulolytic activity may also be determined in endocellulase units (ECU) by measuring the ability of the enzyme to reduce the viscosity of a solution of carboxymethyl cellulose (CMC) .

The ECU assay quantifies the amount of catalytic activity present in the sample by measuring the ability of the sample to reduce the viscosity of a solution of carboxy-methylcellulose (CMC). The assay is carried out at 40°C; pH 7.5; 0.1M phosphate buffer; time 30 min; using a relative enzyme standard for reducing the viscosity of the CMC Hercules 7 LFD substrate; enzyme concentration approx. 0.15 ECU/ml. The arch standard is defined to 8200 ECU/g.

### Colour Measurement

A Nippon Denshoku's spectrophotometer (SE 2000), which was in accordance with JIS Z8722, ASTM E308, ASTM E313 and ASTM D1925, was used according to the manufacturer's instructions to evaluate the chromaticity using the change in the colour space coordinates L*a*b* (CIELAB-system), where as usual:
L* gives the change in white/black on a scale from 0 to 100, and a decrease in L* means an increase in black colour (decrease in white colour) and an increase in L* means an increase in white colour (decrease in black colour).
a* gives the change in red/green, and a decrease in a* means an increase in green colour (decrease in red colour), and an increase in a* means an increase in red colour (decrease in green colour).
b* gives the change in blue/yellow, and a decrease in b* means an increase in blue colour (decrease in yellow colour), and an increase in b* means an increase in yellow colour (decrease in blue colour) (Vide WO 96/12846 NOVO).

The Nippon Denshoku's spectrophotometer (SE 2000) was operated in the L*a*b* colour space. The light source was D65 standard light. The software used for evaluation was ColorMate Version 4.05. The illumination and light-receiving conditions of this instrument is 0-45° after spectrum method based on JIS Z-8722 and was calibrated using the white and black tiles. Each result was an average of 4 measurements. Fabric rinsed without enzyme and mediator was measured and the coordinates L*a*b* were calculated and entered as a reference. The coordinates of the samples were then for each of L*, a*, b* calculated as the difference of the average of the measurements on each swatch from the reference value.

The present invention is further illustrated in the following examples, which are not in any way intended to limit the scope of the invention as claimed.

### EXAMPLE 1

### Comparison of anti-back staining effect between Cutinase A and Endolase

An Indigo solution was prepared by washing denim with model washing agent. The compositions of model washing agent are as follows:
Sodiumdihydrogen phospate: 6.2g/20L
Sodium citrate: 5.8g/20L
Novasol P: 2.4g/20L
Carezyme 1000L (available from Novozymes A/S): 2.8g/20L

The washing conditions were as follows:
Temperature: 55°C
Washing Time: 120 min
Enzyme: Model washing agent
Enzyme dosage: 1g/L
Washing liquor: Deionized water (3°dH) / 20L
Denim: Kurabo KD511
Bath ratio: 1:20
Washing machine: Wascator (FOM71MP-Lab.)

Swatches (10cm x 10cm) of polyester and polyester/cotton was washed with the indigo solution (pH=6.5) prepared above with Cutinase A and a cellulase (Denimax^{®} 362S), respectively. The conditions were:
Temperature: 55°C
Washing Time: 60min
Washing liquor: Indigo solution (pH=6.5)
Enzymes: Cutinase A and Endolase (Novozym^{®} 613, 3090ECU/g)
Enzyme dosage:
   0, 1, 3, 5, 10mg enzyme protein/L
Swatch: Polyester, Polyester/Cotton
Swatch size: 10cm X 10cm
Bath ratio: (Polyester X 2, Polyester/Cotton X 2)/L
T-O-M: 120rpm

### Results:

**Table 1. Comparison of anti-back staining effect between Cutinase A and Endolase (L* value)**

| Enzyme | Textile | 0mg/L* 1 | 1mg/L* 1 | 3mg/L* 1 | 5mg/L* 1 | 10mg/L*1 |
|---|---|---|---|---|---|---|
| Cutinase A | Polyester | 68.4 | 71.6 | 75.7 | 79.6 | 84.0 +/- |
| | | +/- | +/- | +/- | +/- | 0.2 |
| | | 0.8 | 0.2 | 0.2 | 0.4 | |
| | Plyester/Co tton | 71.8 | 71.8 | 73.9 | 74.5 | 76.9 +/- |
| | | +/- | +/- | +/- | +/- | 0.2 |
| | | 0.1 | 0.1 | 0.1 | 0.1 | |
| | Polyester | 68.4 | 69.6 | 68.8 | 69.3 | 68.6 +/- |
| Endolase | | +/- | +/- | +/- | +/- | 0.4 |
| | | 0.8 | 0.3 | 0.5 | 0.4 | |
| | Plyester/Co tton | 71.8 | 72.4 | 72.7 | 72.1 | 72.7 +/- |
| | | +/- | +/- | +/- | +/- | 0.4 |
| | | 0.1 | 0.2 | 0.2 | 0.1 | |

| | | | | | | |
|---|---|---|---|---|---|---|
| *¹ Enzyme protein base | | | | | | |

The above results show a significant anti-back staining effect on polyester and polyester/cotton of the cutinase compared with the cellulase. The cellulase did not show any anti-back staining effect on the fabric swatches.

### EXAMPLE 2

### Anti-back staining effect of Cutinase A and B and Lipolase.

An Indigo solution was prepared by washing denim with Denimax^{®} 362S in deionised water. The conditions were as follows:
Temperature: 55°C
Washing Time: 120min
Enzyme: Denimax^{®} 362S
Enzyme dosage: 1g/L
Washing liquor: Deionized water (3°dH) / 20L
Denim: Kurabo KD511
Bath ratio: 1:20
Washing machine: Wasicator (FOM71MP-Lab)

Swatches (10cm x 10cm) of polyester and polyester/cotton was washed with the indigo solution (pH=6.5) prepared above with the cutinases and Lipolase^{®} 100L (available from Novozymes A/S), respectively. The conditions were:
Temperature: 55°C
Washing Time: 60min
Washing liquor: Indigo solution (pH=6.5)
Enzymes: Cutinase A and B and Lipolase^{®} 100L, type EX
Enzyme dosage: 0, 10, 30, 50 mg enzyme protein/L (Table 2) and 0, 1, 3, 5mg enzyme protein/L (Table 3)
Swatch: Polyester and Polyester/Cotton
Swatch size: 10cm X 10cm
Bath ratio: (Polyester X 2, Polyester/Cotton X 2) /L
T-O-M: 120rpm

### Results:

**Table 2. Anti-back staining effect of enzymes on polyester and polyester/cotton (L* value)**

| Textile | Enzyme | 0mg/L*¹ | 10mg/L*¹ | 30mg/L*¹ | 50mg/L*¹ |
|---|---|---|---|---|---|
| | Cutinase | 65.4 | 84.6 +/- | 89.0 +/- | 89.7 +/- |
| Polyester | A | +/- 0.3 | 0.1 | 0.0 | 0.1 |
| | Cutinase | 65.7 | 88.3 +/- | 90.3 +/- | 90.8 +/- |
| | B | +/- 0.2 | 0.1 | 0.2 | 0.2 |
| | Lipolase | 65.1 | 66.0 +/- | 68.1 +/- | 69.0 +/- |
| | | +/- 0.2 | 0.4 | 0.2 | 0.4 |
| | Cutinase | 67.9 | 76.3 +/- | 83.3 +/- | 84.6 +/- |
| Polyester/Cot | A | +/- 0.3 | 0.2 | 0.0 | 0.1 |
| ton | | | | | |
| | Cutinase | 68.6 | 82.3 +/- | 86.7 +/- | 86.9 +/- |
| | B | +/-0.1 | 0.1 | 0.1 | 0.1 |
| | Lipolase | 68.1 | 69.2 +/- | 71.8 +/- | 73.6 +/- |
| | | +/- 0.3 | 0.1 | 0.1 | 0.2 |

| | | | | | |
|---|---|---|---|---|---|
| *¹ Enzyme protein base | | | | | |

**Table 3. Anti-back staining effect of cutinase with low enzyme dosage (L* value)**

| Textile | Enzyme | 0mg/L*² | 1mg/L*² | 3mg/L*² | 5mg/L*² |
|---|---|---|---|---|---|
| | Cutinas | 63.7 +/- | 68.6 +/- | 75.9 +/- | 81.2 +/- |
| Polyester | e A | 0.1 | 0.5 | 0.6 | 0.4 |
| | Cutinas | 63.9 +/- | 71.0 +/- | 76.3 +/- | 79.3 +/- |
| | e B | 0.2 | 0.1 | 0.3 | 0.6 |
| Polyester/Cotton | Cutinase A | 64.2 +/- 0.1 | 65.5 +/- 0.2 | 67.5 +/- 0.4 | 71.0 +/-0.1 |
| | Cutinase B | 64.5 +/-0.2 | 66.3 +/-0.2 | 69.7 +/-0.4 | 72.2 +/-0.3 |

The above results show an anti-back staining effect on polyester and polester/cotton of the cutinases and the Lipolase.

### EXAMPLE 3

### Anti-back staining effect of cutinase and lipases at acid pH condition

An Indigo solution was prepared by washing denim with Cellusoft^{®} L in deionised water. The conditions were as follows:
Temperature: 55°C
Washing Time: 120min
Enzyme: Cellusoft^{®} L
Enzyme dosage: 1g/L
Buffer: 1M Acetate buffer (pH=4.8) / 100ml/20L
Washing liquor: Deionized water (3°dH) / 20L
Denim: Kurabo KD511
Bath ratio: 1:20
Washing machine: Wasicator (FOM71MP-Lab.)

Swatches (10cm x 10cm) of polyester and polyester/cotton was washed with the indigo solution (pH=5) prepared above with Cutinase A and B, Lipolase^{®} and Lipolase^{™} Ultra, respectively.
The conditions were:
Temperature: 55°C
Washing Time: 60min
Washing liquor: Indigo solution (pH=5)
Enzymes: Cutinase A and B, Lipolase^{®} and Lipolase^{™} Ultra
Enzyme dosage: 0, 10, 30, 50 and 100mg enzyme protein/L (Table 4) and 0, 10 and 30 mg enzyme protein/L (Table 5)
Swatch: Polyester, Polyester/Cotton
Swatch size: 10cm X 10cm
Bath ratio: (Polyester X 2, Polyester/Cotton X 2)/L
T-O-M: 120rpm

### Results:

**Table 4. Anti-back staining effect of Cutinase A and B (L* value)**

| Enzyme | Textile | 0mg/L | 10mg/L | 30mg/L | 50mg/L | 100mg/L |
|---|---|---|---|---|---|---|
| | | *¹ | *¹ | *¹ | *¹ | *¹ |
| | Polyester | 68.8+ | 74.7+/ | 79.6+/ | 79.9+/ | 82.2+/- |
| Cutina -se A | | /-0.5 | -1.0 | -0.2 | -0.1 | 1.6 |
| | | | | | | |
| | Plyester/Co tton | 65.3+ | 66.8+/ | 68.1+/ | 67.5+/ | 69.9+/- |
| | | /-0.9 | -0.2 | -0.6 | -1.0 | 0.6 |
| | Polyester | 66.7+ | 80.3+/ | 82.4+/ | 82.7+/ | 81.7+/- |
| Cutina -se B | | /-0.3 | -0.2 | -0.6 | -0.2 | 0.6 |
| | | | | | | |
| | Plyester/Co tton | 67.4+ | 69.2+/ | 69.2+/ | 70.6+/ | 70.8+/- |
| | | /-0.9 | -0.4 | -1.0 | -0.1 | 0.5 |

| | | | | | | |
|---|---|---|---|---|---|---|
| *¹ Enzyme protein | | | | | | |

### Results:

**Table 5. Anti-back staining effect of Cutinase A and B, Lipolase^{®} and Lipolase^{™} Ultra (L* value)**

| Textile | Enzymes | 0mg/L*¹ | 10mg/L*¹ | 30mg/L*¹ |
|---|---|---|---|---|
| | Cutinase A | 68.5+/- | 77.9+/- | 81.9+/- |
| Polyester | | 0.4 | 0.5 | 0.5 |
| | Cutinase B | Ditto | 79.6+/- | 81.5+/- |
| | | | 0.9 | 0.3 |
| | Lipolase^{®} | Ditto | 80.0+/- | 81.5+/- |
| | | | 0.8 | 0.9 |
| | Lipolase^{™} | Ditto | 75.5+/- | 75.6+/- |
| | Ultra | | 0.4 | 0.4 |
| | Cutinase A | 62.0+/- | 62.6+/- | 63.7+/- |
| Polyester/Cotto n | | 0.8 | 0.6 | 0.6 |
| | | | | |
| | Cutinase B | Ditto | 63.8+/- | 64.3+/- |
| | | | 0.5 | 0.7 |
| | Lipolase^{®} | Ditto | 63.5+/- | 67.3+/- |
| | | | 0.5 | 0.2 |
| | Lipolase^{™} | Ditto | 63.2+/- | 65.2+/- |
| | Ultra | | 0.5 | 0.6 |

The above results show an anti-back staining effect of the cutinases and the lipases on polyester and polyester/cotton at acidic pH.

## Claims

1. A method for reducing the backstaining of fabric or textile selected from the group consisting of polyamide fibres, acrylic fibres, and polyester fibres, comprising contacting the fabric or textile with a composition comprising an effective amount of a cutinase (EC 3.1.1.74).

2. A method for reducing the backstaining of fabric or textile, comprising contacting the fabric or textile with a composition comprising an effective amount of a microbial cutinase (EC 3.1.1.74).

3. The method of claims 1 or 2, wherein the cutinase is a fungal cutinase.

4. The method according to claims 1 or 2, wherein the enzyme is derived from a strain of *Humicola insolens.*

5. The method according to claim 2, wherein the fabric or textile is polyester or polyester/cotton, preferably polyester or polyester/cotton parts of indigo dyed denim.

6. The method according to claims 1 or 2, wherein the amount of enzyme is 1-100 mg of enzyme protein per 1 of composition.

7. The method according to claims 1 or 2, wherein the pH is 4.5-7.5 and the temperature is 40-60 °C.

8. The method according to claims 1 or 2 for simultaneous reducing the backstaining and formation of localized colour variation, wherein the composition further contains a cellulase and/or pumice.

9. Use of a composition comprising a cutinase (EC 3.1.1.74) and a cellulase for reducing backstaining of fabric or textile.

10. The use of claim 9, further comprising a surfactant.

11. The use of claim 9, wherein the cutinase is microbial, preferably of fungal origin, especially preferred derived from a strain of *Humicola insolens.*

## Patentansprüche

1. Verfahren zur Verminderung der Rückfärbung eines Stoffs oder eines Textils, ausgewählt aus der Gruppe bestehend aus Polyamid-Fasern, Acryl-Fasern und Polyester-Fasern, das das in Kontakt bringen des Stoffs oder des Textils mit einer Zusammensetzung umfasst, die eine effektive Menge einer Cutinase (EC 3.1.1.74) aufweist.

2. Verfahren zur Verminderung der Rückfärbung eines Stoffs oder eines Textils, das das in Kontaktbringen des Stoffs oder des Textils mit einer Zusammensetzung umfasst, die eine effektive Menge einer mikrobiellen Cutinase (EC 3.1.1.74) aufweist.

3. Verfahren nach Anspruch 1 oder 2, wobei die Cutinase eine Pilzcutinase ist.

4. Verfahren nach Anspruch 1 oder 2, wobei das Enzym von einem Stamm von *Humicola insolens* stammt.

5. Verfahren nach Anspruch 2, wobei der Stoff oder das Gewebe Polyester oder Polyester/Baumwolle ist, vorzugsweise Polyester- oder Polyester/Baumwoll-Teile von mit Indigo gefärbtem Denim.

6. Verfahren nach Anspruch 1 oder 2, wobei die Menge des Enzyms 1 bis 100 mg des Enzymproteins pro 1 Zusammensetzung beträgt.

7. Verfahren nach Anspruch 1 oder 2, wobei der pH 4,5 - 7,5 und die Temperatur 40 - 60 °C beträgt.

8. Verfahren nach Anspruch 1 oder 2 für die simultane Verminderung der Rückfärbung und der Bildung lokalisierter Farbvariationen, wobei die Zusammensetzung weiterhin eine Cellulase und/oder Bims enthält.

9. Verwendung einer Zusammensetzung, die eine Cutinase (EC 3.1.1.74) und eine Cellulase umfasst, für die Verminderung der Rückfärbung eines Stoffs oder eines Textils.

10. Verwendung nach Anspruch 9, weiterhin umfassend ein Surfaktant.

11. Verwendung nach Anspruch 9, wobei die Cutinase mikrobiellen Ursprungs, vorzugsweise von pilzlichem Ursprung, ist, besonders bevorzugt von einem Stamm von *Humicola insolens* stammt.

## Revendications

1. Méthode pour diminuer la recoloration d'une étoffe ou d'un tissu sélectionné parmi le groupe consistant en des fibres polyamides, des fibres acryliques et des fibres polyesters, comprenant la mise en contact de l'étoffe ou du tissu avec une composition comprenant une quantité efficace d'une cutinase (EC 3.1.1.74).

2. Méthode pour diminuer la recoloration d'une étoffe ou d'un tissu, comprenant la mise en contact de l'étoffe ou du tissu avec une composition comprenant une quantité efficace d'une cutinase microbienne (EC 3.1.1.74).

3. Méthode selon les revendications 1 ou 2, dans laquelle la cutinase est une cutinase fongique.

4. Méthode selon les revendications 1 ou 2, dans laquelle l'enzyme est dérivée d'une souche *d'Humicola insolens.*

5. Méthode selon la revendication 2, dans laquelle l'étoffe ou le tissu est un polyester ou un polyester/coton, de préférence les parties polyester ou polyester/coton d'un denim coloré par l'indigo.

6. Méthode selon les revendications 1 ou 2, dans laquelle la quantité d'enzyme est de 1-100 mg de protéine d'enzyme par 1 de composition.

7. Méthode selon les revendications 1 ou 2, dans laquelle le pH est de 4,5-7,5 et la température est de 40-60°C.

8. Méthode selon les revendications 1 ou 2 pour diminuer simultanément la recoloration et la formation de variation de couleur localisée, dans laquelle la composition contient en outre une cellulase et/ou une ponce.

9. Utilisation d'une composition comprenant une cutinase (EC 3.1.1.74) et une cellulase pour diminuer la recoloration d'une étoffe ou d'un tissu.

10. Utilisation selon la revendication 9, comprenant en outre un tensioactif.

11. Utilisation selon la revendication 9, dans laquelle la cutinase est microbienne, de préférence d'origine fongique, de manière particulièrement préférée d'une souche d*'Humicola insolens.*
